# EUROPÄISCHE PATENTSCHRIFT

(11) **EP 4 051 110 B1**
(45) Veröffentlichungstag und Bekanntmachung des Hinweises auf die Patenterteilung: **01.11.2023**
(21) Anmeldenummer: 20781459.1
(22) Anmeldetag: 24.09.2020
(51) Int. Cl.: A61B 5/347, A61B 5/00, A61N 1/37

(54) **VORRICHTUNG SOWIE VERFAHREN ZUR DETEKTION EINES INNERHALB EINES INDIVIDUUMS BEFINDLICHEN MEDIZINISCHEN AKTIVEN IMPLANTATS**
DEVICE AND METHOD FOR DETECTING A MEDICALLY ACTIVE IMPLANT IMPLANTED WITHIN A PERSON
DISPOSITIF ET PROCÉDÉ DE DÉTECTION D'UN IMPLANT ACTIF MÉDICAL SITUÉ À L'INTÉRIEUR D'UN INDIVIDU

(30) Priorität: 30.10.2019 DE 102019216780
(43) Veröffentlichungstag der Anmeldung: 07.09.2022
(73) Patentinhaber: Neuroloop GmbH, 79110 Freiburg (DE)
(72) Erfinder: PLACHTA, Dennis, 79110 Freiburg (DE); KÖNNE, Nils, 79110 Freiburg (DE)
(74) Vertreter: Rösler, Uwe
(86) Internationale Anmeldenummer: PCT/EP2020/076734
(87) Internationale Veröffentlichungsnummer: WO 2021/083586

(56) Entgegenhaltungen:
- WO-A2-2008/035070
- US-B2- 7 907 992
- JURCO JURAJ ET AL: "Precise pacing artefact detection", 2016 COMPUTING IN CARDIOLOGY CONFERENCE (CINC), CCAL, 11. September 2016 (2016-09-11), Seiten 761-764, XP033071273, DOI: 10.22489/CINC.2016.220-375 [gefunden am 2017-03-01]
- HELFENBEIN E ET AL: "An automated algorithm for the detection of atrial fibrillation in the presence of paced rhythms", COMPUTERS IN CARDIOLOGY, 2010, IEEE, 26. September 2010 (2010-09-26), Seiten 113-116, XP032008236, ISBN: 978-1-4244-7318-2
- KUMAR ASHISH ET AL: "Efficient QRS complex detection algorithm based on Fast Fourier Transform", BIOMEDICAL ENGINEERING LETTERS, THE KOREAN SOCIETY OF MEDICAL AND BIOLOGICAL ENGINEERING, KOREA, Bd. 9, Nr. 1, 25. Oktober 2018 (2018-10-25), Seiten 145-151, XP036737986, ISSN: 2093-9868, DOI: 10.1007/S13534-018-0087-Y [gefunden am 2018-10-25]
- MOND HARRY G ET AL: "The Footprints of Electrocardiographic Interference: Fact or Artefact", HEART, LUNG AND CIRCULATION, ELSEVIER, AMSTERDAM, NL, Bd. 28, Nr. 10, 12. April 2019 (2019-04-12), Seiten 1472-1483, XP085804586, ISSN: 1443-9506, DOI: 10.1016/J.HLC.2019.03.006 [gefunden am 2019-04-12]

## Beschreibung

### Technisches Gebiet

Die Erfindung bezieht sich auf eine Vorrichtung sowie ein Verfahren zur Detektion eines innerhalb eines Individuums befindlichen medizinischen aktiven, zur elektrischen Stimulation befähigten Implantats. Ferner wird eine Verwendung der Vorrichtung zur Bestimmung des Betriebszustandes des medizinisch aktiven Implantats erläutert.

In immer mehr Bereichen der Medizin werden aktive Implantate zur Behandlung verschiedener Erkrankungen eingesetzt. Im Unterschied zu rein passiven Implantaten, die typischerweise eine rein mechanische Stütz- und Tragefunktion besitzen, umfassen aktive Implantate elektronische Komponenten, die zur Unterstützung von Organfunktionen elektrische Signale zumeist zu detektieren sowie zu Stimulationszwecken zu erzeugen und lokal zu applizieren in der Lage sind.

Das Vorhandensein eines solchen aktiven Implantats lässt daher auf das Vorhandensein der entsprechenden klinischen Indikation schließen. Im Falle einer Notfallsituation des Patienten, kann die Kenntnis über die klinische Indikation für das implantierte aktive Implantat eine Veränderung der Notfallbehandlung nötig machen. Aus diesem Grund ist es wichtig, dass das medizinische Personal auch in Situationen, in denen ein Patient selbst keine Auskunft mehr geben kann, von einem vorhandenen, aktiven Implantat erfährt.

### Stand der Technik

Patienten, die ein medizinisch aktives Implantat tragen, bekommen nach Artikel 18 Abs. 2 der europäischen Medizinprodukteverordnung einen Implantationsausweis ausgehändigt, mit welchem das entsprechende Implantat identifiziert werden kann.

Somit ist die Information über das Implantat nur verfügbar, solange der Patient diese noch Kommunizieren kann und/oder der Ausweis mitgeführt wird.

Die Druckschriften US 2006/ 0 293 714 A1, US 4 291 703 A, US 5 406 955 A sowie US 9 788 756 B2 offenbaren jeweils Vorrichtungen und Verfahren zur Detektion eines medizinischen Implantats.

Die Druckschrift WO 2008/035070 A2 beschreibt ein Verfahren zur Erzeugung einer Dominanzfrequenzkarte eines Herzbereichs eines Patienten. Dabei wird die Stimulation des rechten Ventrikels des Patienten elektrokardiographisch gemessen und ein Leistungsfrequenzspektrum nach schneller Fourier-Transformation ermittelt.

### Darstellung der Erfindung

Der Erfindung liegt die Aufgabe zugrunde eine Vorrichtung sowie ein Verfahren zur Detektion eines innerhalb eines Individuums befindlichen medizinischen aktiven, zur elektrischen Stimulation befähigten Implantats anzugeben, so dass auf nicht invasivem Wege schnell, zuverlässig und ohne die Notwendigkeit einer Kommunikation mit dem Individuum festgestellt werden kann, ob ein aktives Implantat vorhanden ist und falls ja, um welche Art von Implantat es sich handelt. Insbesondere soll es möglich sein, ein nachgewiesenes Implantat anhand seines Stimulationsmusters zu identifizieren und dessen Betriebszustand zu beurteilen.

Die Lösung der der Erfindung zugrunde liegenden Aufgabe ist im Anspruch 1 angegeben. Gegenstand der Ansprüche 12 und 13 richten sich auf eine Verwendung der Vorrichtung zur Identifikation und Bestimmung des Betriebszustandes des Implantats. Gegenstand des Anspruches 14 ist ein lösungsgemäßes Verfahren.

Den Erfindungsgedanken in vorteilhafter Weise weiterbildende Merkmale sind Gegenstand der Unteransprüche sowie der Beschreibung, insbesondere unter Bezugnahme auf die illustrierten Ausführungsbeispiele zu entnehmen.

Die lösungsgemäße Vorrichtung zur Detektion eines innerhalb eines Individuums befindlichen medizinischen aktiven, zur elektrischen Stimulation befähigten Implantats verfügt über wenigstens zwei mit dem Individuum in elektrischen Kontakt stehenden oder bringbaren Elektroden, einer elektrischen Spannungsmesseinheit, die mit den wenigstens zwei Elektroden elektrisch verbunden ist und elektrische Spannungszeitsignale zu erzeugen vermag, sowie eine mit der elektrischen Spannungsmesseinheit verbundene Auswerteeinheit, mit der es möglich ist, auf Basis der gemessenen elektrischen Spannungszeitsignale jene Information abzuleiten, die die Gegenwart sowie die Art bzw. den Typ eines aktiven Implantats belegen. Hierzu umfasst die Auswerteeinheit eine Einheit zur Ermittlung eines Leistungsdichtespektrums basierend auf den zwischen den wenigstens zwei Elektroden gemessenen elektrischen Spannungszeitsignalen, vorzugsweise in Form eines Spektrumanalysators. Zudem dienen eine Einheit zur Bestimmung von lokalen Maxima innerhalb des Leistungsdichtespektrums und deren jeweils zugeordnete, sogenannte Peakfrequenz, sowie eine Einheit zur Ermittlung eines kleinsten gemeinsamen Nenners unter den bestimmten Peakfrequenzen. Schließlich umfasst die Auswerteeinheit eine Komparatoreinheit, die auf der Grundlage des ermittelten kleinsten gemeinsamen Nenners, der einer Stimulationsfrequenz des medizinischen aktiven Implantats entspricht und in einer Referenzdatenbank bevorrateten Informationen einen Datenvergleich ausführt und ein Vergleichsergebnis generiert, von dem abhängig eine mit der Komparatoreinheit in Kommunikation stehende Signaleinheit ein Signal erzeugt.

Das der lösungsgemäßen zugrunde liegende Messprinzip besteht im Wesentlichen darin, zwischen wenigstens zwei definiert vorgegebenen Hautoberflächenbereichen des Individuums eine elektrische Spannungsmessung durchzuführen und das dabei erhaltene elektrische Spannungssignal zeitlich und spektral zu analysieren. Neben der Verwendung von auf der Haut eines Individuums anbringbaren Klebeelektroden eignen sich gleichsam auch mittels temporären Unterdrucks anhaftende Elektroden, wie sie üblicherweise in Verbindung mit Standard-EKG-Geräten eingesetzt werden.

Für die elektrische Spannungsmessung geeignete Kontaktbereiche stellen vorzugsweise Kopf-, Hals-, Oberkörper-, Leisten- oder Beinbereiche dar, insbesondere vorzugsweise längs der bekannten Äquipotentialflächen des Erregungspotentials des Herzens

Die wenigstens zwei am Individuum applizierten Elektroden sind galvanisch, d.h. leitungsgestützt mit der elektrischen Spannungsmesseinheit verbunden, die als separates Bauteil oder als integraler Bestandteil innerhalb einer Baueinheit untergebracht ist, die zudem eine aus mehreren Komponenten zur spektralen Untersuchung und Auswertung der erfassten elektrischen Spannungszeitsignale bestehenden Auswerteeinheit umfasst. Die Baueinheit kann als Moduleinsatz in bestehenden medizinischen Diagnoseapparaturen integriert oder als portable Einheit für den manuellen Feldeinsatz konzipiert sein.

Die mit Hilfe der elektrischen Spannungsmesseinheit erfassten elektrischen Spannungszeitsignale werden mit einer Abtastfrequenz fs von wenigstens 100 Hz, vorzugsweise mit Abtastfrequenzen zwischen 1 MHz und 1 Ghz, erfasst und vorzugsweise innerhalb einer Speichereinheit abgespeichert. In der Speichereinheit oder in einer weiteren Speichereinheit sind zudem für einen Datenvergleich bevorratete Informationen in Form einer Referenzdatenbank abgespeichert.

Zur digitalen Signalverarbeitung und Abspeicherung der analogen von Seiten der Spannungsmesseinheit bereitgestellten elektrischen Spannungszeitsignale werden die analogen Spannungszeitsignale mit Hilfe eines Analog-Digital-Wandlers in digitale Signale gewandelt, die der Auswerteeinheit zugeführt werden, deren einzelne Komponenten im Wesentlichen auf Basis digitaler Signalprozessoren und/oder Mikrocontroller realisiert sind.

So sieht die Auswerteeinheit als erste Komponente eine Einheit zur Ermittlung eines Leistungsdichtespektrums vor, das die spektrale Leistungsdichte der auf die Frequenz bezogenen Leistung des elektrischen Spannungszeitsignals innerhalb des möglichen Frequenzbereiches zwischen 0 und fs12 repräsentiert.

Nach der Ermittlung des Leistungsdichtespektrums werden in einer zweiten Komponente, der sogenannten Einheit zur Bestimmung lokaler Maxima, die spektrale Lage der lokalen Maxima innerhalb des Leistungsdichtespektrums bestimmt, die jeweils durch eine so genannte Peakfrequenz charakterisiert ist. Ein mögliches Kriterium für die Bestimmung von lokalen Maxima ist beispielsweise, dass die spektrale Leistungsdichte innerhalb einer bestimmten Frequenzbreite, z.B. 2 Hz, um die Peakfrequenz herum um z.B. 3 dB abgenommen hat.

Unter den vorstehend ermittelten Peakfrequenzen wird im Weiteren mit einer dritten Komponente der Auswerteeinheit, der sogenannten Einheit zur Ermittlung des kleinsten gemeinsamen Nenners der kleinste gemeinsame Nenner zumindest unter den meisten der ermittelten Peakfrequenzen bestimmt, der einer oder der Stimulationsfrequenz eines medizinischen aktiven Implantats innerhalb des Individuums entspricht. Die Stimulationsfrequenz sowie auch die Amplituden aller Harmonischen der Stimulationsfrequenz werden in der Speichereinheit für eine weitere Verarbeitung und einen weiteren Informationszugriff abgespeichert.

Ist eine Ermittlung eines kleinsten gemeinsamen Nenners unter den bestimmten Peakfrequenzen nicht möglich, so kann dies als Hinweis darauf gewertet werden, dass im Individuum kein aktives Implantat vorhanden ist oder ein vorhandenes Implantat nicht aktiv bzw. nicht funktionsfähig ist. In diesem Fall generiert die Einheit zur Ermittlung eines kleinsten gemeinsamen Nenners ein Signal, das in entsprechend codierter Form durch eine Signaleinheit, vorzugsweise auf einem Display darstellbar ist, um einen Arzt oder Nutzer der Vorrichtung entsprechend zu informieren.

Kann hingegen ein kleinster gemeinsamer Nenner unter den bestimmten Peakfrequenzen ermittelt werden, so gilt dies als Hinweis auf die Existenz eines aktiven Implantats, das mit der gemessenen Stimulationsfrequenz intrakorporal stimuliert. Mit Hilfe einer in der Baueinheit vorgesehenen weiteren Komponente, der sogenannten Komparatoreinheit, wird wenigstens auf der Grundlage der ermittelten Stimulationsfrequenz sowie mit in einer Referenzdatenbank bevorrateten Informationen ein Datenvergleich durchgeführt, der zu einem Vergleichsergebnis führt, in dessen Abhängigkeit ein mit Hilfe der Signaleinheit optisch, akustisch oder haptisch wahrnehmbares Signal, generiert wird, wodurch ein Nutzer nicht nur über die Präsenz eines aktiven Implantates und dessen Betriebszustand, sondern darüber hinaus auch über dessen Art bzw. Typ und damit den möglichen, zugrunde liegenden, klinischen Indikationen informiert wird. Vorzugsweise ist die Signaleinheit als Display zur Darstellung alphanumerischer Zeichen ausgebildet.

Durch geeignete Kategorisierungen von in der Referenzdatenbank hinterlegten Informationen, bspw. nach Fehler-freien oder Fehler-behafteten Zuständen sowie von Definitionen von allgemeinen oder Implantat-spezifischen Toleranzbereichen, können bei der nachgewiesenen Präsenz eines medizinischen aktiven Implantats zusätzlich Aussagen über dessen Betriebszustand getroffen werden.

Um die Belastbarkeit der Art- bzw. Typbestimmung zu dem unbekannten Implantat zu verbessern, werden in vorteilhafter Weise weitere Informationen herangezogen, die aus den gemessenen und gegebenenfalls abgespeicherten elektrischen Spannungszeitsignalen ableitbar sind und mit entsprechenden in der Referenzdatenbank bevorrateten Informationen mittels der Komparatoreinheit verglichen. Beispielsweise eignen als zusätzliche Informationen der gesamte Verlauf des gemessenen Spannungszeitsignals sowie daraus entnehmbare bzw. ableitbare Informationen über die Stimulationspulseigenschaften des medizinischen aktiven Implantat, bspw. die Stimulationspulsform, Stimulationspulsweite, Stimulationspulssymmetrie, Stimulationspulsanzahl, Stimulationspulsamplitude und zeitliche Abfolge sowie zeitliche Korrelation der Stimulationspulse relativ zum Herzschlag.

Auch können aus dem zeitlichen Verlauf des Leistungsdichtespektrums im Bereich der festgestellten Stimulationsfrequenz sowie deren höheren Harmonischen jeweils charakteristische Stimulationsmuster extrahiert und mit geeigneten Referenzdaten verglichen werden. Handelt es sich bei dem aktiven Implantat beispielsweise um einen Herzschrittmacher, so zeichnet sich das diesbezügliche Stimulationsmuster durch kurze Stimulationspulse aus, die mit dem Herzschlag synchronisiert sind. Betrifft das aktive Implantat hingegen einen Stimulator zum Zwecke der Blutdrucksenkung, so zeichnen sich die diesbezüglichen Stimulationssignale durch zeitlich eher kontinuierlich in Erscheinung tretende Stimulationssignale aus. In einer weiteren bevorzugten Ausführungsform ist die elektrische Spannungseinheit mit wenigstens drei Elektroden elektrisch verbunden, die an unterschiedlichen Bereichen an der Hautoberfläche eines Individuums platziert sind. Durch die sich daraus ergebende Möglichkeit der Messung von wenigstens drei elektrischen Spannungszeitsignalen kann im Wege einer Triangulationsmessung eine Ortsbestimmung des medizinischen, aktiven Implantats innerhalb des Individuums vorgenommen werden. Die Auswerteeinheit umfasst hierzu eine Triangulationseinheit, die auf Basis von zwischen den wenigstens drei Elektroden gemessenen elektrischen Spannungen eine Ortsbestimmung des medizinischen aktiven Implantats durchführt.

Vorzugsweise sind sämtliche Komponenten der Auswerteeinheit, d.h. die Einheit zur Ermittlung des Leistungsdichtespektrums, die Einheit zur Bestimmung lokaler Maxima innerhalb des Leistungsdichtespektrums, die Einheit zur Ermittlung des kleinsten gemeinsamen Nenners, die Einheit zur Ermittlung der Stimulationspulseigenschaften sowie die Komparatoreinheit in Form von digitalen Signalprozessoren oder Mikrocontrollern realisiert, deren Funktionalität jeweils durch Software basierte Auswerte- und Bestimmungsregeln vorgegeben ist.

Eine weitere Ausführungsform der lösungsgemäßen Vorrichtung sieht eine Schnittstelle vor, über die elektrische Spannungszeitsignale von einem EKG-Gerät direkt der Auswerteeinheit zugeleitet werden können und zur Detektion und Identifikation eines sich möglicherweise innerhalb eines Individuums befindlichen aktiven Implantates dienen. In diesem Fall sind die Elektroden Teil des EKG-Gerätes. Auch ist es denkbar, dass die lösungsgemäße Vorrichtung durch entsprechende Ergänzung durch eine EKG-Messeinheit die vollständige Messung und Erfassung eines Elektrokardiogramms eines Individuums übernimmt, so dass während der Durchführung eines Standard-EKG-Monitorings die Detektion und Identifizierung möglicher aktiver Implantate auf Basis der EKG-Spannungszeitsignale durchgeführt werden kann.

### Kurze Beschreibung der Erfindung

Die Erfindung wird nachstehend ohne Beschränkung des allgemeinen Erfindungsgedankens anhand eines Ausführungsbeispiels unter Bezugnahme auf die Zeichnungen exemplarisch beschrieben. Es zeigt:

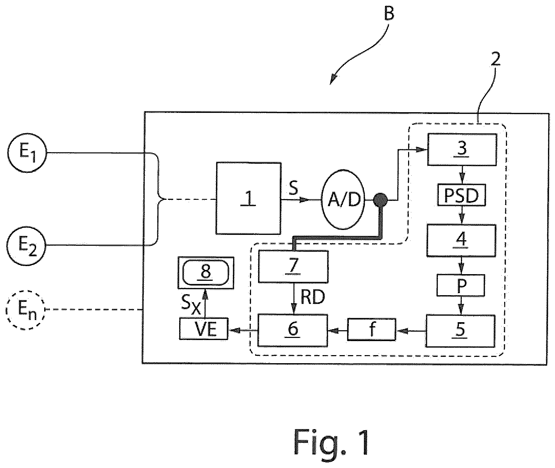

- Fig. 1: Schematisierte Darstellung einer lösungsgemäßen Vorrichtung zur Detektion und Identifikation eines aktiven medizinischen Implantats.

### Wege zur Ausführung der Erfindung, gewerbliche Verwendbarkeit

Figur 1 zeigt eine Baueinheit B, die in Form einer modulartigen Einschubeinheit in ein Medizinrack oder als portabel handhabbare Funktionseinheit ausgeführt sein kann.

Die Vorrichtung ist mit wenigstens zwei Elektroden E1, E2 verbunden, kann jedoch beliebig auf n-Elektroden erweitert werden, die jeweils zur lösbar festen Anbringung an der Hautoberfläche einer Person ausgebildet sind.

Zur Erfassung der elektrischen Spannung zwischen den Elektroden E1, E2 ist in der Baueinheit B eine Spannungsmesseinheit 1 vorgesehen, die die elektrische Potentialdifferenz zwischen den Elektroden E1, E2 zeitaufgelöst erfasst und elektrische Spannungszeitsignale S erzeugt. Die analogen elektrischen Spannungszeitsignale S werden mit Hilfe eines Analogdigitalwandlers A/D in digitale Spannungszeitsignale konvertiert, die zur weiteren Auswertung bzw. Bewertung sowohl der Auswerteeinheit 2, die Teil der Baueinheit B ist, als auch der Speichereinheit (7) zugeführt werden.

Innerhalb der Auswerteeinheit 2 ist eine Einheit 3 zur Ermittlung des Leistungsdichtespektrums vorgesehen, in der prozessorgestützt eine Spektralanalyse der gemessenen elektrischen Spannungszeitsignale S zum Erhalt eines Leistungsdichtespektrums PSD durchgeführt wird. Ferner umfasst die Auswerteeinheit 2 eine Einheit 4 zur Bestimmung lokaler Maxima innerhalb des Leistungsdichtespektrums PSD, denen jeweils eine so genannte Peakfrequenz P zugeordnet ist. In einer weiteren Einheit 5 wird zu den bestimmten bzw. ermittelten Peakfrequenzen P ein kleinster gemeinsamer Nenner ermittelt, der der so genannten Stimulationsfrequenz f entspricht.

Im Rahmen einer Komparatoreinheit 6 wird zum Erhalt eines Vergleichsergebnisses VE ein Vergleich auf der Grundlage von in einer Speichereinheit 7 bevorrateten Referenzdaten RD und zumindest der ermittelten Stimulationsfrequenz f durchgeführt. Optional können der Komparatoreinheit 6 zur Vergleichsdurchführung weitere Informationen bereitgestellt werden, die sich aus den elektrischen Spannungszeitsignalen S bzw. aus den digitalisierten Spannungszeitsignalen extrahieren lassen, wie bspw. die Pulsform der Stimulationspulse des medizinischen, aktiven Implantats, deren Pulssymmetrie, Pulsweite, Pulsamplitude, Pulsanzahl, zeitliche Pulsabfolge, sowie deren zeitliche Korrelation zum Herzschlag. Zu den weiteren Informationen sind entsprechende Referenzdaten zum Vergleich in der Speichereinheit hinterlegt.

Die Komparatoreinheit 6 generiert in Abhängigkeit des Vergleichsergebnisses VE ein Signal Sx, das einer Signaleinheit 8 zugeführt wird, die in Abhängigkeit des Signals S_{X} visuell, akustisch und/oder haptisch wahrnehmbar in Erscheinung tritt. Vorzugsweise stellt die Signaleinheit 8 ein Display dar, anhand dem die Information über ein aufgefundenes aktives Implantat und dessen Betriebszustand, sowie zugrundeliegender, möglicher klinischer Indikationen in Form alphanumerischer Zeichen darstellbar ist.

### Bezugszeichenliste

- B: Baueinheit
- 1: Spannungsmesseinheit
- 2: Auswerteeinheit
- 3: Einheit zur Ermittlung eines Leistungsdichtespektrums
- 4: Einheit zur Bestimmung von lokalen Maximal innerhalb des Leistungsdichtespektrums
- 5: Einheit zur Ermittlung eines kleinsten gemeinsamen Nenners der bestimmten Peakfrequenzen
- 6: Komparatoreinheit
- 7: Speichereinheit
- 8: Signaleinheit
- PSD: Leistungsdichtespektrum
- P: Peakfrequenz
- f: Stimulationsfrequenz
- RD: Referenzdaten
- VE: Vergleichsergebnis
- S_{X}: Ergebnissignal

## Patentansprüche

1. Vorrichtung zur Detektion eines innerhalb eines Individuums befindlichen medizinischen aktiven, zur elektrischen Stimulation befähigten Implantats mit
- wenigstens zwei mit dem Individuum in elektrischen Kontakt bringbaren Elektroden,
- einer elektrischen Spannungsmesseinheit (1), die mit den wenigstens zwei Elektroden elektrisch verbunden ist und elektrische Spannungszeitsignale zu erzeugen vermag,
- einer mit der elektrischen Spannungsmesseinheit (1) verbundenen Auswerteeinheit (2) umfassend die nachfolgenden Komponenten:
- eine Einheit (3) zur Ermittlung eines Leistungsdichtespektrums (PSD) basierend auf den zwischen den wenigstens zwei Elektroden gemessenen elektrischen Spannungszeitsignalen,
- eine Einheit (4) zur Bestimmung von lokalen Maxima innerhalb des Leistungsdichtespektrums (PSD) und deren jeweils zugeordneten, sogenannten Peakfrequenz (P),
- eine Einheit (5) zur Ermittlung eines kleinsten gemeinsamen Nenners von zumindest einem Teil der bestimmten Peakfrequenzen (P),
- eine Komparatoreinheit (6), die auf der Grundlage des ermittelten kleinsten gemeinsamen Nenners, der einer Stimulationsfrequenz (f) des medizinischen aktiven Implantats entspricht, und in einer Referenzdatenbank bevorrateter Informationen (RD) einen Datenvergleich ausführt und ein Vergleichsergebnis (VE) generiert, sowie
- eine mit der Komparatoreinheit (6) in Kommunikation stehenden Signaleinheit (8), die in Abhängigkeit des Vergleichsergebnisses (VE) ein Signal erzeugt.

2. Vorrichtung nach Anspruch 1,
**dadurch gekennzeichnet, dass** die Elektroden als Hautkontaktelektroden ausgebildet sind.

3. Vorrichtung nach Anspruch 1 oder 2,
**dadurch gekennzeichnet, dass** die Elektroden EKG-Standardelektroden sind.

4. Vorrichtung nach einem der Ansprüche 1 bis 3,
**dadurch gekennzeichnet, dass** die Einheit (3) zur Ermittlung des Leistungsdichtespektrums (PSD) ein Spektrumanalysator ist.

5. Vorrichtung nach einem der Ansprüche 1 bis 4
**dadurch gekennzeichnet, dass** die elektrische Spannungseinheit (1) mit einem A/D-Wandler zum Erhalt digitalisierter elektrischer Spannungszeitsignale verbunden ist, dessen Ausgang mit der in Form wenigstens eines digitalen Signalprozessors oder Mikrocontrollers ausgebildeten Auswerteeinheit (3) verbunden ist.

6. Vorrichtung nach Anspruch 5,
**dadurch gekennzeichnet, dass** die Auswerteeinheit (3) eine Speichereinheit (7) umfasst, in der zumindest die digitalisierten elektrischen Spannungszeitsignale abspeicherbar sind.

7. Vorrichtung nach einem der Ansprüche 1 bis 6,
**dadurch gekennzeichnet, dass** die Auswerteeinheit (3) eine Einheit zur Bestimmung von Informationen umfasst, die aus den elektrischen Spannungszeitsignalen ableitbar sind und Stimulationspulse betreffen, die vermittels des medizinischen aktiven Implantats erzeugbar sind, und wenigstens eine Information der nachfolgenden Informationen umfasst: Stimulationspulsform, Stimulationspulssymmetrie, Stimulationspulsweite, Stimulationspulsamplitude, Stimulationspulsanzahl, zeitliche Abfolge der Stimulationspulse, sowie zeitliche Korrelation von Stimulationspulsen zum Herzschlag.

8. Vorrichtung nach Anspruch 6 oder 7,
**dadurch gekennzeichnet, dass** die Komparatoreinheit (6) die in der Speichereinheit (7) abgespeicherten elektrischen Spannungszeitsignale und/oder die hiervon abgeleiteten Informationen mit den in der Referenzdatenbank abgespeicherten Informationen (RD) vergleicht, die Spannungszeitsignalen und/oder hiervon abgeleitete Informationen bekannter medizinischer Implantate entsprechen.

9. Vorrichtung nach einem der Ansprüche 6 bis 8,
**dadurch gekennzeichnet, dass** die Komparatoreinheit (6) einen Funktionsmustervergleich zwischen den abgespeicherten Spannungszeitsignalen und/oder den hiervon abgeleiteten Informationen und den Spannungszeitsignalen und/oder hiervon abgeleiteten Informationen bekannter medizinischer Implantate durchführt.

10. Vorrichtung nach einem der Ansprüche 1 bis 9,
**dadurch gekennzeichnet, dass** die Signaleinheit (8) eine visuell und/oder akustisch wahrnehmbare Signaleinheit ist.

11. Vorrichtung nach einem der Ansprüche 1 bis 10,
**dadurch gekennzeichnet, dass** die elektrische Spannungsmesseinheit (1) mit wenigstens drei Elektroden elektrisch verbunden ist, und
dass die Auswerteeinheit (2) eine Triangulationseinheit umfasst, die auf Basis von zwischen den wenigstens drei Elektroden gemessenen elektrischen Spannungen eine Ortsbestimmung des medizinischen aktiven Implantats durchführt.

12. Verwendung der Vorrichtung zur Detektion eines unbekannten aktiven medizinischen Implantats nach einem der Ansprüche 1 bis 11 zu dessen Identifikation.

13. Verwendung der Vorrichtung nach einem der Ansprüche 1 bis 11 zur Bestimmung des Betriebszustandes eines aktiven medizinischen Implantats durch Vergleich der in der Speichereinheit (7) abgespeicherten elektrischen Spannungszeitsignale und/oder der hiervon abgeleiteten Informationen mit in der Referenzdatenbank abgespeicherten kategorisierten Informationen (RD), betreffend Fehler-freie oder Fehler-behaftete Zustände des Implantats, Definitionen von allgemeinen oder Implantat-spezifischen Toleranzbereichen.

14. Verfahren zur Detektion eines in einem Individuum befindlichen medizinischen aktiven Implantats **gekennzeichnet durch** die Kombination folgender Verfahrensschritte:
- Durchführen einer elektrischen Spannungsmessung mit Hilfe wenigstens zweier mit dem Individuum in elektrischem Kontakt stehenden Elektroden jeweils zum Erhalt von elektrischen Spannungszeitsignalen,
- Durchführen einer Spektralanalyse der gemessenen elektrischen Spannungszeitsignale zum Erhalt eines Leistungsdichtespektrums,
- Bestimmen von lokalen Maxima innerhalb des Leistungsdichtespektrums (PSD) und deren jeweils zugeordnete, sogenannte Peakfrequenz (P),
- Ermitteln eines kleinsten gemeinsamen Nenners von den bestimmten Peakfrequenzen (P), der einer Stimulationsfrequenz des medizinischen aktiven Implantats entspricht,
- Vergleichen der Stimulationsfrequenz mit Referenzdaten (RD) zum Erhalt eines Vergleichsergebnisses (VE) sowie
- Identifizieren des medizinischen aktiven Implantats auf der Grundlage des Vergleichsergebnisses (VE).

15. Verfahren nach Anspruch 14,
**dadurch gekennzeichnet, dass** auf der Grundlage der elektrischen Spannungszeitsignalen Informationen abgeleitet werden, die Stimulationspulse betreffen, die vermittels des medizinischen aktiven Implantats erzeugt werden, und wenigstens eine Information der nachfolgenden Informationen umfasst:
Stimulationspulsform, Stimulationspulssymmetrie, Stimulationspulsweite, Stimulationspulsamplitude, Stimulationspulsanzahl, zeitliche Abfolge der Stimulationspulse, sowie zeitliche Korrelation von Stimulationspulsen zum Herzschlag, und
dass die von den Spannungszeitsignalen abgeleiteten Informationen mit Referenzdaten (RD) zum Erhalt eines Vergleichsergebnisses (VE) verglichen werden, das zur Identifizierung des medizinischen Implantats und/oder zur Bestimmung des Betriebszustandes des aktiven medizinischen Implantats verwendet wird.

## Claims

1. A device for the detection of a medically active implant capable of electrical stimulation that is located within an individual and comprises
- at least two electrodes that can be brought into electrical contact with the individual,
- an electrical voltage measuring unit (1), which is electrically connected to the at least two electrodes and is able to produce electrical voltage time signals,
- an evaluation unit (2) that is connected to the electrical voltage measuring unit (1) and comprises the following components:
- a unit (3) for determining a power spectral density (PSD) based on the electrical voltage time signals measured between the at least two electrodes,
- a unit (4) for determining local maxima within the power spectral density (PSD) and the so-called peak frequency (P) respectively assigned to them,
- a unit (5) for determining the lowest common denominator of at least one part of the determined peak frequencies (P),
- a comparator unit (6), which on the basis of the lowest common denominator, which corresponds to a stimulation frequency (f) of the medically active implant, and information (RD) stored in a reference database, carries out a data comparison and generates a comparator result (VE), as well as
- a signal unit (8) which is in communication with the comparator unit (6) and produces a signal as a function of the comparison result (VE).

2. The device according to claim 1,
**characterised in that** the electrodes are designed as main contact electrodes.

3. The device according to claim 1 or 2,
**characterised in that** the electrodes are ECG standard electrodes.

4. The device according to any one of claims 1 to 3,
**characterised in that** the unit (3) for determining the power spectral density (PSD) is a spectrum analyser.

5. The device according to any one of claims 1 to 4,
**characterised in that** the electrical voltage unit (1) is connected to an A/D converter for obtaining digitalised electrical voltage time signals, the output of which is connected to an evaluation unit (3) configured in the form of at least one digital signal processor or microcontroller.

6. The device according to claim 5,
**characterised in that** the evaluation unit (3) comprises a storage unit (7) in which at least the digitalised electrical voltage time signals can be stored.

7. The device according to any one of claims 1 to 6, **characterised in that** the evaluation unit (3) comprises a unit for determining information that can be derived from the electrical voltage time signals and relates to stimulation pulses that can be produced by way of the medically active implant and includes at least one piece of information from the following information: stimulation pulse shape, stimulation pulse symmetry, stimulation plus width, stimulation pulse amplitude, stimulation pulse number, time sequence of the stimulation pulses, as well as the time correlation of simulation pulses to heartbeat.

8. The device according to claim 6 or 7,
**characterised in that** the comparator unit (6) compares the electrical voltage time signals stored in the storage unit (7) and/or the information derived therefrom, with the information (RD) stored in the reference database, which corresponds to voltage time signals and/or information derived therefrom, of known medical implants.

9. The device according to any one of claims 6 to 8,
**characterised in that** the comparator unit (6) carries out a function pattern comparison between the stored voltage time signals and/or information derived therefrom, and the voltage time signals and/or information derived therefrom, of known medical implants.

10. The device according to any one of claims 1 to 9,
**characterised in that** the signal unit (8) is a visually and/or acoustically perceptible signal unit.

11. The device according to any one of claims 1 to 10, **characterised in that** the electrical voltage measuring unit (1) is electrically connected to at least three electrodes, and **in that** the evaluation unit (2) comprises a triangulation unit, which, on the basis of electrical voltages measured between that at least three electrodes, localises the position of the medically active implant.

12. Use of the device for the detection of an unknown active medical implant in accordance with any one of claims 1 to 11.

13. Use of the device according to any one of claims 1 to 11 to determine the operational status of an active medical implant through comparison of the electrical voltage time signals stored in the storage unit (7) and/or the information derived therefrom, with categorised information (RD) stored in the reference database that relates to fault-free or faulty states of the implant, definitions of general or implant-specific tolerances ranges.

14. A method of detection of a medically active implant located in an individual, **characterised by** the combination of the following process stages:
- carrying out an electrical voltages measurement with the aid of at least two electrodes that are in contact with the individual, each for obtaining electrical voltage time signals,
- carrying out a spectral analysis of the measured electrical voltage signals to obtain a power spectral density,
- determination of local maxima within the power spectral density (PSD) and the so-called peak frequency (P) respectively assigned to them,
- determination of the lowest common denominator of the determined peak frequencies (P) that corresponds to a stimulation frequency of the medically active implant,
- comparison of the simulation frequency with reference data (RD) in order to obtain a comparison value (VE) as well as
- identification of the medically active implant on the basis of the comparison result (VE).

15. The method according to claim 14,
**characterised in that** on the basis of the electrical voltage time signals, information is derived relating to stimulation pulses which are produced through the medically active implant, and includes at least one piece of information from the following information: stimulation pulse shape, stimulation pulse symmetry, stimulation plus width, stimulation pulse amplitude, stimulation pulse number, time sequence of the stimulation pulses, as well as the time correlation of simulation pulses to heartbeat.

## Revendications

1. Dispositif de détection d'un implant actif médical capable de stimulation électrique, situé à l'intérieur d'un individu avec
- au moins deux électrodes pouvant être mises en contact électrique avec l'individu,
- une unité de mesure de tension électrique (1), qui est reliée électriquement à au moins deux électrodes et peut produire des signaux temporels de tension électriques,
- une unité d'évaluation (2) reliée à l'unité de mesure de tension électrique (1) comprenant les composants suivants :
- une unité (3) destinée à déterminer une intensité spectrale de puissance (ISP) en se basant sur les signaux temporels de tension électriques mesurés entre au moins deux électrodes,
- une unité (4) destinée à déterminer des maxima locaux à l'intérieur de l'intensité spectrale de puissance (ISP) et de ladite fréquence de pic (P) respectivement attribuée à ceux-ci,
- une unité (5) destinée à déterminer un plus petit dénominateur commun d'au moins une partie des fréquences de pic (P) déterminées,
- une unité de comparaison (6), qui sur la base du plus petit dénominateur commun déterminé, qui correspond à une fréquence de stimulation (f) de l'implant actif médical et exécute une comparaison de données dans une banque de données de référence des informations stockées (RD) et génère un résultat de comparaison (RC),
et
- une unité de signalisation (8) en communication avec l'unité de comparaison (6), qui produit un signal en fonction du résultat de comparaison (RC).

2. Dispositif selon la revendication 1,
**caractérisé en ce que** les électrodes sont constituées comme des électrodes de contact cutanées.

3. Dispositif selon la revendication 1 ou 2,
**caractérisé en ce que** les électrodes sont des électrodes standard d'électrocardiogramme (ECG).

4. Dispositif selon l'une quelconque des revendications 1 à 3,
**caractérisé en ce que** l'unité (3) est un analyseur spectral destiné à déterminer l'intensité spectrale de puissance (ISP).

5. Dispositif selon l'une quelconque des revendications 1 à 4,
**caractérisé en ce que** l'unité de tension électrique (1) est reliée à un convertisseur analogique/numérique (A/N) pour obtenir des signaux temporels de tension électriques numérisés, dont la sortie est reliée à une unité d'évaluation (3) constituée sous la forme d'au moins un processeur numérique de signaux ou un microcontrôleur.

6. Dispositif selon la revendication 5,
**caractérisé en ce que** l'unité d'évaluation (3) comprend une unité de mémoire (7) dans laquelle peuvent être mémorisées des signaux temporels de tension électrique numérisés.

7. Dispositif selon l'une quelconque des revendications 1 à 6,
**caractérisé en ce que** l'unité d'évaluation (3) comprend une unité destinée à déterminer des informations qui peuvent être déduites des signaux temporels de tension électriques et concernent des impulsions de stimulation, qui peuvent être produites au moyen de l'implant actif médical et comprend au moins une information des informations suivantes : forme d'impulsion de stimulation, symétrie d'impulsion de stimulation, ampleur d'impulsion de stimulation, amplitude d'impulsion de stimulation, nombre d'impulsions de stimulation, succession dans le temps des impulsions de stimulation ainsi que corrélation dans le temps des impulsions de stimulation par rapport au rythme cardiaque.

8. Dispositif selon la revendication 6 ou 7,
**caractérisé en ce que** l'unité de comparaison (6) compare les signaux temporels de tension électriques mémorisés dans l'unité de mémoire (7) et/ou les informations déduites de ceux-ci aux informations (RD) mémorisées dans la banque de données de référence, qui correspondent à des signaux temporels de tension et/ou à des informations déduites de ceux-ci d'implants médicaux connus.

9. Dispositif selon l'une quelconque des revendications 6 à 8,
**caractérisé en ce que** l'unité de comparaison (6) exécute une comparaison type fonctionnelle entre les signaux temporels de tension électriques mémorisés et/ou les informations déduites de ceux-ci et les signaux temporels de tension et/ou les informations déduites de ceux-ci d'implants médicaux connus.

10. Dispositif selon l'une quelconque des revendications 1 à 9,
**caractérisé en ce que** l'unité de signalisation (8) est une unité de signalisation visuellement et/ou acoustiquement décelable.

11. Dispositif selon l'une quelconque des revendications 1 à 10,
**caractérisé en ce que** l'unité de mesure de tension (1) est reliée électriquement à au moins trois électrodes et
**en ce que** l'unité d'évaluation (2) comprend une unité de triangulation, qui effectue une détermination localisée de l'implant actif médical sur la base des tensions électriques mesurées entre au moins trois électrodes.

12. Utilisation du dispositif de détection d'un implant actif médical inconnu selon l'une quelconque des revendications 1 à 11 à des fins d'identification de celui-ci.

13. Utilisation du dispositif selon l'une quelconque des revendications 1 à 11 pour déterminer l'état de fonctionnement d'un implant actif médical par comparaison des signaux temporels de tension électriques mémorisés dans l'unité de mémoire (7) et/ou des informations déduites de ceux-ci à des informations (RD) catégorisées mémorisées dans la banque de données de référence concernant les états sans défaut ou associés à des défauts de l'implant, des définitions des plages de tolérances générales ou spécifiques à l'implant.

14. Procédé de détection d'un implant actif médical situé à l'intérieur d'un individu **caractérisé par** la combinaison des étapes de procédé suivantes :
- exécution d'une mesure de tension électrique à l'aide d'au moins deux électrodes en contact électriques avec l'individu pour obtenir respectivement des signaux temporels de tension,
- exécution d'une analyse spectrale des signaux temporels de tension électriques mesurés pour obtenir une intensité spectrale de puissance,
- détermination de maxima locaux à l'intérieur de l'intensité spectrale de puissance (ISP) et de ladite fréquence de pic (P) respectivement attribuée à ceux-ci,
- détermination d'un plus petit dénominateur commun des fréquences de pic déterminées (P), qui correspond à une fréquence de stimulation de l'implant actif médical,
- comparaison de la fréquence de stimulation aux données de référence (RD) pour obtenir un résultat de comparaison (RC) et
- identification de l'implant actif médical sur la base du résultat de comparaison (RC).

15. Procédé selon la revendication 14,
**caractérisé en ce que** des informations sont déduites sur la base des signaux temporels de tension, qui concernent des impulsions de stimulation, qui sont produites au moyen de l'implant actif médical et comprend au moins une information des informations suivantes :
forme d'impulsion de stimulation, symétrie d'impulsion de stimulation, ampleur d'impulsion de stimulation, amplitude d'impulsion de stimulation, nombre d'impulsions de stimulation, succession dans le temps des impulsions de stimulation ainsi que corrélation dans le temps des impulsions de stimulation par rapport au rythme cardiaque, et
**en ce que** les informations déduites des signaux temporels de tension sont comparées à des données de référence (RD) pour obtenir un résultat de comparaison (RC), qui est utilisé pour identifier l'implant médical et/ou pour déterminer l'état de fonctionnement de l'implant actif médical.
